# EUROPEAN PATENT APPLICATION

(11) **EP 3 329 988 A1**
(43) Date of publication of application: **06.06.2018**
(21) Application number: 17780292.3
(22) Date of filing: 28.02.2017
(51) Int. Cl.: B01F 13/08, C12N 1/00, C12N 5/00

(54) **STIRRER, STIRRING DEVICE, STIRRING METHOD, CELL CULTURING METHOD, REACTION-PROMOTING METHOD, AND METHOD FOR ASSEMBLING STIRRER**

(30) Priority: 18.04.2016 JP 2016082808
(71) Applicant: Isel Co., Ltd., Yao-shi, Osaka 581-0068 (JP)
(72) Inventor: MOCHIZUKI Noboru, Yao-shi Osaka 581-0068 (JP)
(74) Representative: Gunzelmann, Rainer
(86) International application number: PCT/JP2017/008011
(87) International publication number: WO 2017/183320

(57) **Abstract**

There are provided a stirring unit, a stirring device, a stirring method, and a method of assembling a stirring unit which make it possible to efficiently and rapidly stir a fluid within a vessel.

A stirring unit 1 stirs a fluid A contained in a vessel 6. Stirring unit 1 includes a mixing body 2 for stirring fluid A by rotating around a rotation axis S, magnets 5a and 5b or magnetic substances for receiving a rotating magnetic field for rotating mixing body 2. A suction port 20α and a discharge port 20β of fluid A are disposed on a surface of mixing body 2, and one or more holes 22 connecting suction port 20α and discharge port 20β are disposed within mixing body 2, suction port 20α is disposed at a position on rotation axis S or a position closer to rotation axis S than discharge port 20β, and discharge port 20β is disposed at a position outside rotation axis S than suction port 20α.

## Description

### TECHNICAL FIELD

The present invention relates to a stirring unit, a stirring device, a stirring method, a cell cultivation method, a reaction promoting method, and a method of assembling a stirring unit for stirring a fluid contained in a vessel.

### BACKGROUND ART

Conventionally, in order to stir a fluid contained in a vessel, there is well known a combination of a magnetic stirrer and a stirring unit (see Patent Literature 1 and Patent Literature 2 below). The stirring unit is placed so as to be submerged in a bottom part of the vessel, and rotates by receiving a rotational magnetic force from the magnetic stirrer to stir the fluid in the vessel. As the conventional stirring unit, there is a rotating body which is a stirring bar or a disk having a cross-shaped protrusion with magnets at ends of the disk, or a structure having a stirring blade provided on a rotating body (see Patent Literature 1 below).

### CITATION LIST

### Patent Literature

Patent Literature 1: Publication of Japanese Patent Application No. 2007-136443
Patent Literature 2: Publication of Japanese Patent Application No. 2006-150221

### SUMMARY OF INVENTION

### TECHNICAL PROBLEM

However, in a conventional stirring unit, in order to stir a fluid in a vessel, there is a problem that it is difficult to stir the fluid located in upper portion of the vessel above an upper part of a center of the stirring unit having a small tangential speed. This is because, the fluid at an outer peripheral portion of the vessel is easy to be stirred by a large force of rotation transmitted from end portions of the stirring unit causing large turbulence of the flow of the fluid, while the fluid at an upper central portion of the vessel is difficult to be stirred due to a small force of rotation transmitted from the central portion of the stirring unit causing small turbulence of the flow of the fluid. In addition, in the vertical direction within the vessel, the force transmitted from the stirring unit is larger toward the bottom side closer to the stirring unit but smaller toward the upper side due to viscosity of the fluid. As a result, the fluid in the vessel is stirred most quickly at the outer peripheral portion of the bottom of the vessel, and the fluid in an upper central portion of the vessel is stirred most slowly. As described above, with the conventional stirring unit, stirring of the fluid in the vessel proceeds from the outer peripheral portion of the vessel to the central portion of the vessel and also proceeds from the vessel bottom to the upper portion of the vessel. Therefore, it takes a relatively long time for uniformly stirring the entire fluid in the vessel.

The present invention has been made in view of the above circumstances, and it is an object of the present invention to provide a stirring unit, a stirring device, and a stirring method capable of efficiently and rapidly stirring a fluid in a vessel. Another object of the present invention is to provide a cell cultivation method using the stirring unit, a reaction promoting method, and a method of assembling the stirring unit.

### SOLUTION TO PROBLEM

According to the present invention, there is provided a stirring unit for stirring a fluid contained in a vessel including a mixing body for stirring the fluid by rotating around a rotation axis, and a magnet for receiving a rotating magnetic field for rotating the mixing body, wherein a suction port and a discharge port for the fluid are provided on a surface of the mixing body, one or two or more holes connecting the suction port and the discharge port are provided within the mixing body, the suction port is disposed at a position on the rotation axis or at a position closer to the rotation axis than the discharge port, and the discharge port is disposed at a position outside the rotation axis than the suction port.

According to the above configuration, as the stirring unit disposed in the vessel is rotated, the fluid within the mixing body is caused to flow out from the discharge port to the outside by a force generated from rotation. Then, the fluid in the vicinity of the central upper portion in the vessel is sucked into the mixing body from the suction port of the mixing body. As a result, the fluid in the outer peripheral portion of the stirring unit is mixed by being disturbed by the fluid flowing out from the discharge port. As described above, it is possible to suck the fluid at the center upper part in the vessel from the suction port of the mixing body without retention and allow the fluid to flow out from the discharge port, so that the fluid can be efficiently stirred and the time for uniformly mixing the entire fluid in the vessel can be shortened.

Further, according to the present invention, there is provided a stirring device including the stirring unit and a rotating magnetic field generating unit for generating a rotating magnetic field for rotating the stirring unit.

Furthermore, according to the present invention , there is provided a stirring method including; disposing a stirring unit at a bottom of a vessel; rotating the stirring unit at a center of the bottom within the vessel containing the fluid; sucking the fluid at a central upper portion in the vessel from a suction port by the stirring unit; passing the fluid within the stirring unit; and flowing out the fluid from the discharge port of the stirring unit to an outer peripheral portion within the vessel to stir the fluid.

The present invention also relates to a cell cultivation method in which a fluid in a vessel is used as a cell cultivation medium and the cell cultivation solution is stirred with the stirring unit, or a reaction promoting method in which a fluid in the vessel is used as a reaction solution and the reaction solution is stirred with the stirring unit thereby promoting the reaction.

Further, according to the present invention, there is provided a method for assembling a stirring unit including a step of forming a mixing body and a step of fixing the mixing body to a base having a magnet or a magnetic substance, wherein the step of forming the mixing body includes a step of aligning and stacking a plurality of mixing elements to form the mixing body, and the step of fixing the mixing body to the base has a step of fixing the mixing body to the base by penetrating the mixing body in the stacking direction by a fixing member.

### ADVANTAGEOUS EFFECTS OF INVENTION

According to the present invention, since a large fluid flow occurs in the vertical direction of the vessel, the fluid in the vessel can be rapidly mixed. Therefore, it is possible to shorten the mixing time of the fluid.

### BRIEF DESCRIPTION OF DRAWINGS

[Fig. 1] A cross-sectional view showing a stirring device using a stirring unit according to an embodiment of the present invention;
[Fig. 2] A perspective view and a side view showing the stirring unit;
[Fig. 3] A perspective view and side views showing a base of the stirring unit;
[Fig. 4] A plan view showing a configuration of mixing elements constituting a mixing body of the stirring unit;
[Fig. 5] A partial plan view and a cross-sectional view of the mixing body showing a fluid flow state within the same;
[Fig. 6] A plan view showing a configuration of mixing elements according to a modification 1 of the mixing body;
[Fig. 7] A perspective view of mixing elements according to a modification 2 of the mixing body and a sectional view showing a fluid flow state in the mixing elements;
[Fig. 8] A perspective view of mixing elements according to a modification 3 of the mixing body and a partial sectional view showing a sectional shape of the mixing elements;
[Fig. 9] Sectional views of the respective mixing bodies according to a modification 4 of the mixing body;
[Fig. 10] Perspective views of the respective bases according to modifications of the base;
[Fig. 11] A perspective view and a plan view of a base of another example according to a modification of the base;
[Fig. 12] A perspective view of a stirring unit according to other embodiment 1 of the present invention;
[Fig. 13] Perspective views showing other examples of the stirring unit of the other embodiment 1;
[Fig. 14] Perspective views of stirring units according to other embodiment 2 of the present invention;
[Fig. 15] Perspective views showing other examples of the stirring unit of the other embodiment 2;
[Fig. 16] Perspective views of stirring units according to other embodiment 3 of the present invention;
[Fig. 17] Perspective views showing an example of a stirring unit according to other embodiment 4 of the present invention;
[Fig. 18] Perspective views of a stirring unit according to other embodiment 4 of the present invention;
[Fig. 19] A perspective view of a stirring unit according to other embodiment 5 of the present invention;
[Fig. 20] Photographs showing stirring units used in examples and comparative examples;
[Fig. 21] Photographs illustrating a state of stirring in Examples 1 and 2; and
[Fig. 22] Photographs illustrating a state of stirring in Comparative Examples 1 and 2.

### DESCRIPTION OF EMBODIMENTS

Embodiments of the present invention will be described hereinafter with reference to the accompanying drawings.

As to directions described in the present specification, "stacking direction" is synonymous with the direction of the rotation axis of a stirring unit 1 (including a mixing body 2 and a base 3), the vertical direction and the like, and "extending direction" is synonymous with the direction orthogonal to the stacking direction, the radial direction and the circumferential direction of mixing elements 21.

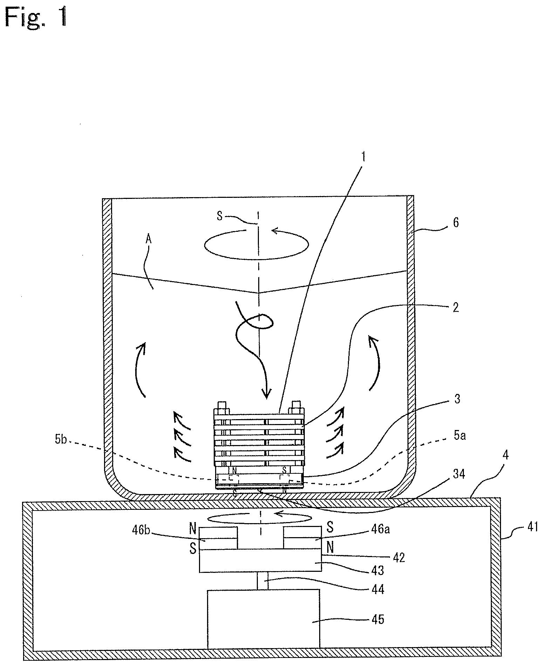

Fig. 1 is a cross-sectional view of a stirring device according to an embodiment of the present invention, which includes a stirring unit 1 and a magnetic stirrer 4 for generating a rotating magnetic field for rotating stirring unit 1.

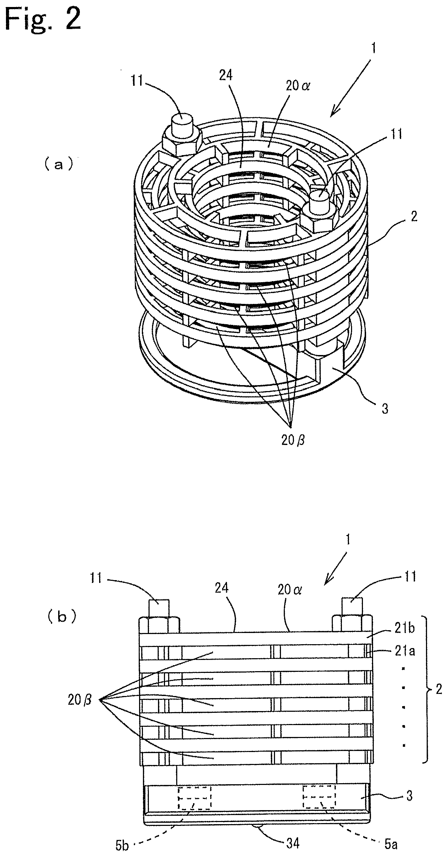

Stirring unit 1 is disposed in a vessel 6 such as a beaker, and rotated by receiving a rotating magnetic field from magnetic stirrer 4 on which vessel 6 is placed to stir a fluid A such as a sample contained in vessel 6. It should be noted that the present invention is not limited to the case where stirring unit 1 is disposed in vessel 6 such as the beaker and used for experiments and the like, but may be arranged in vessel 6 such as a tank for containing fluid A such as various raw materials including being used for commercial use. Fig. 2 at (a) and Fig. 2 at (b) show a perspective view and a side view of stirring unit 1. Referring to the figures, there are shown a mixing body 2 for stirring fluid A by rotating about a rotation axis S, and a base 3 incorporating magnets 5a and 5b for supporting mixing body 2 and receiving a rotating magnetic field. Although permanent magnets are used for magnets 5a and 5b, magnetic substances may be used instead of them (this also applies to embodiments other than this embodiment).

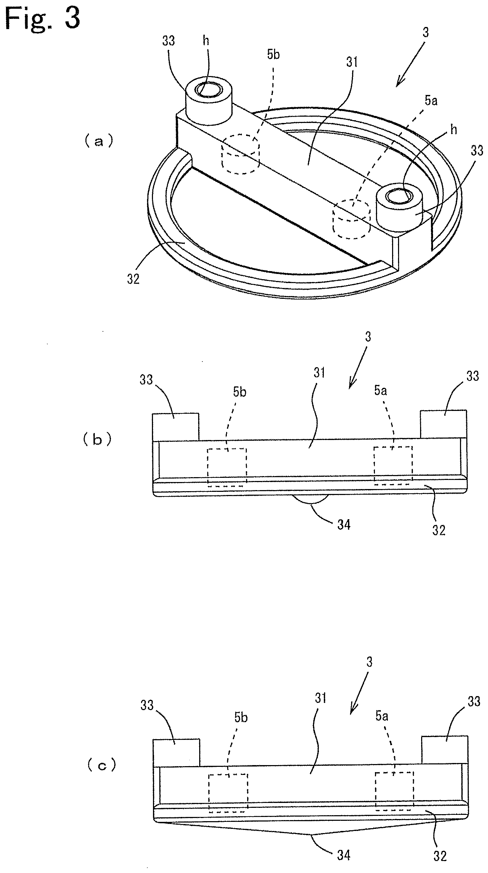

Fig. 3 at (a) and Fig. 3 at (b) respectively show a perspective view and a side view of base 3 which is provided with a pillar-shaped bar body 31 that is horizontally placed. At both ends of an upper surface of bar body 31, there are provided screw cylinder portions 33 for mounting and fixing mixing body 2, respectively. At both ends of bar body 31, there are also respectively accommodated magnets 5a and 5b which receive the rotating magnetic field from magnetic stirrer 4 of Fig. 1. The respective magnets 5a and 5b are arranged so that the magnetization direction faces the vertical direction orthogonal to the longitudinal direction of bar body 31 and also the positions of the magnetic poles of magnets 5a and 5b are opposite to each other. As shown in Fig. 1, in the horizontally placed state of bar body 31, for example, when the N pole of one magnet 5a is disposed on its lower surface side, the S pole of other magnet 5b is disposed on its lower surface side. Ring, cylindrical or rectangular magnets may be used as magnets 5a and 5b, but a bar magnet having such a size as to be housed over the longitudinal direction of bar body 31 may be housed within bar body 31.

In addition, bar body 31 is provided with an annular portion 32 for preventing stirring unit 1 from falling. Annular portion 32 is connected to both end portions of bar body 31 so as to be flush with a lower surface of bar body 31. An arcuate support protrusion 34 to be in contact with a bottom surface of vessel 6 is protruded at a center of the lower surface of bar body 31. As a result, since support protrusion 34 serves as a center of rotation, stirring unit 1 is supported almost without shifting during rotation. Therefore, the frictional resistance between stirring unit 1 and the bottom surface of vessel 6 becomes extremely small, so that stirring unit 1 can be rotated smoothly.

If desired, two or more arc-shaped stabilizing protrusions may be provided at equal intervals with a height equal to or less than the protrusion height of support protrusion 34 on the lower surface of annular portion 32 (including the connected portion of bar body 31). In this case, even if the attitude of stirring unit 1 supported by support protrusion 34 at the time of rotation is inclined obliquely due to the weight of mixing body 2 or the like, the stabilizing protrusions contact the bottom surface of vessel 6 to keep it in a stable attitude. Fig. 3 at (c) shows a side view of a base 3 as a modification, in which support protrusion 34 may be formed over the entire lower surface of bar body 31.

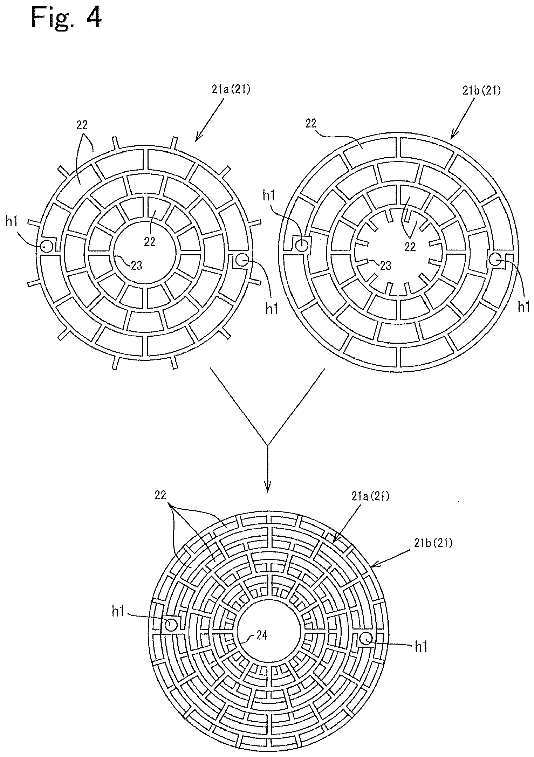

As shown in Fig. 2 at (a) and Fig.2 at (b), mixing body 2 is constituted by a stacked structure in which a plurality of mixing elements 21 (here, 10 elements) having a disk or substantially disk shape are stacked, and the stacked mixing elements 21are fastened to screw cylinder portions 33 of base 3 (see Fig. 3 at (a)) to be fixed to base 3 by inserting bolts 11 (fixing members) through bolt holes "h1" (see Fig. 4) provided at two locations at 180 degrees apart in an outer peripheral portion of the elements. Thus, mixing body 2 in which the plurality of mixing elements 21 are stacked and disassemblably integrated is easily constructed and fixed to base 3. Further, by constituting the plurality of mixing elements 21 that can be disassembled into each element, it is possible to easily carry out a cleaning operation such as removal of residues and foreign matters remaining in each disassembled mixing element 21 (first through holes 22, second through holes 23 and the like shown in Fig. 4). The structure for integrating the plurality of mixing elements 21 and the structure for attaching mixing body 2 to base 3 are not limited to fixing with bolts 11 but also may be a disengageable attachment structure such as a concave and convex fitting structure.

As shown in Fig. 4, mixing body 2 is formed by alternately stacking two kinds of mixing elements 21a and 21b, and these two kinds of mixing elements 21a and 21b each have a plurality of first through holes 22 penetrating in the thickness direction. Mixing elements 21a and 21b shown in Fig. 2 have different numbers of partition walls in the circumferential direction and the radial direction from those shown in Fig. 4, but the other structures are common. The plurality of first through holes 22 are provided along surfaces extending in the extending direction of the disk-shaped mixing elements 21a and 21b. Each of mixing elements 21a and 21b at its central portion is provided with a second through hole 23 having an opening area larger than that of first through hole 22. Second through hole 23 is formed in a substantially circular shape. As shown in Fig. 2 at (a), a cylindrical hollow portion 24 in which second through holes 23 communicate with each other is formed by stacking mixing elements 21a and 21b. An upper opening of hollow portion 24 constitutes a suction port 20α of fluid A (see Fig. 1). The central axis of hollow portion 24 coincides with rotation axis S of stirring unit 1 of Fig. 1. Therefore, suction port 20α is arranged at a position on rotation axis S.

Further, as shown in Fig. 4, first through holes 22 are formed in a substantially rectangular shape as seen in plan view, and arranged concentrically around the center point of second through hole 23. First through holes 22 are arranged in a staggered manner, and each arrangement pattern itself of first through holes 22 is different in two kinds of mixing elements 21a and 21b. In mixing element 21a, first through holes 22 are closed on an inner peripheral surface facing second through hole 23 and open on an outer peripheral surface of element 21a, while in other mixing element 21b first through holes 22 are open on an inner peripheral surface facing second through hole 23 and closed on an outer peripheral surface of element 21b. As shown in Fig. 2, first through holes 22 opened to the outer peripheral surfaces of mixing elements 21a constitute discharge ports 20β of fluid A (see Fig. 1). Accordingly, discharge ports 20β are disposed at positions outside the rotation axis S (see Fig. 1) than suction port 20α which is the upper end opening of hollow portion 24 (that is, at a position outside in the radial direction orthogonal to rotation axis S).

First through holes 22 are formed to be the same in size in the same circumferential directions of mixing elements 21a and 21b, and formed so as to become larger toward the outside of the mixing elements 21a and 21b in the radial directions. Further, in the overlapping state of the two types of mixing elements 21a and 21b, each area of a portion where first through holes 22 overlap with each other is the same in the circumferential direction.

First through holes 22 of the adjacent mixing elements 21a and 21b in mixing body 2 are arranged so as to partially overlap with each other in the radial direction and the circumferential direction, and communicate with each other in the stacking direction and the extending direction of mixing elements 21a and 21b. In other words, the respective partition walls between first through holes 22 extending in the radial direction and in the circumferential direction of mixing elements 21a and 21b are disposed with mutually different positions between the adjacent mixing elements 21a and 21b. Accordingly, an inner portion of mixing body 2 is formed such that fluid A is sequentially passed between first through holes 22 of adjacent mixing elements 21and 21b so that fluid A is divided in the stacking direction and the extending direction of mixing elements 21a and 21b. Thus, as shown in Fig. 2, the plurality of first through holes 22 within mixing body 2 form a plurality of "flow paths" for connecting an upper opening of hollow portion 24 serving as suction port 20α and first through holes 22 opened to the outer circumferential surface of mixing element 21a serving as discharge port 20β.

Mixing elements 21a and 21b and base 3 constituting mixing body 2 are made of a resin such as polyethylene, polypropylene, fluorine resin or the like, but may be made of ceramic, metal, or the like. By making mixing elements 21a and 21b and base 3 made of resin, it is possible to easily and inexpensively manufacture mixing elements 21a and 21b and base 3 by resin molding. Further, in the case where the mixing elements 21a and 21b and the base 3 are made of a resin other than the fluorine-based resin, the surface layer may be formed of a fluorine-based resin by coating or the like. In this case, the chemical resistance is improved and it can be preferably used also in the field of mixing chemical agents and the like.

As a method of assembling stirring unit 1 of Fig. 2, there are provided a step of forming mixing body 2 and a step of fixing mixing body 2 to base 3.

In the step of forming mixing body 2, a plurality of mixing elements 21a and 21b are aligned at predetermined positions in the circumferential direction and are alternately stacked to form mixing body 2 as shown in Fig. 4. In this case, since the bolt holes "hl" to be inserted by bolts 11 (fixing member) are provided at a pair of outer peripheral portions of mixing elements 21a and 21b, the respective mixing elements 21a and 21b are stacked by inserting bolts 11 through bolt holes "hl" of the outer peripheral portion, whereby the elements can be easily aligned at predetermined positions in the circumferential direction. In the case where bolt holes "hl" of mixing elements 21a and 21b are located closer to the center or are provided at the center portion, the elements can be efficiently aligned at a predetermined position in the circumferential direction by bringing a jig or the like into contact with the outer peripheral portion. In the step of fixing mixing body 2 to base 3, mixing body 2 stacked by the plurality of mixing elements 21a and 21b is penetrated by bolts 11 in the stacking direction, and fixed by screwing bolts 11 into screw holes "h" of the base 3. Through the above steps, the plurality of mixing elements 21a and 21b are aligned in predetermined positions in the circumferential direction to form the stacked mixing body 2, and the attachment to base 3 is completed, so that the assembly of stirring unit 1 is efficiently performed.

Meanwhile, as shown in Fig. 1, magnetic stirrer 4 includes a main body 41, an upper surface of which is horizontal, and a rotating magnetic field generating section 42 arranged within main body 41. Rotating magnetic field generating section 42 includes a plate-shaped driving rotator 43 extending in the horizontal direction and driving magnets 46a and 46b provided on both upper end portions of a driving rotator 43, respectively. Each of driving magnets 46a and 46b is disposed such that the magnetization direction faces in a direction (vertical direction) perpendicular to the extending surface (horizontal plane) of driving rotator 43, and the magnetic poles (N pole and S pole) are arranged so that their positions are opposite to each other. A motor 45 is connected to a central portion of a lower surface of driving rotator 43 via a shaft portion 44. By rotation driving of motor 45, driving rotator 43 and the pair of driving magnets 46a and 46b are rotated in the horizontal direction so that a rotating magnetic field is generated on an upper surface of main body 41.

Next, a method of stirring fluid A using stirring unit 1 having the above-described configuration will be described.

As shown in Fig. 1, vessel 6 containing fluid A is placed on the upper surface of main body 41 of magnetic stirrer 4, stirring unit 1 is placed on the bottom surface of vessel 6, and the magnetic force generated from magnets 46a and 46b attracts magnets 5a and 5b of base 3 of stirring unit 1. Then, by driving and rotating motor 45 of magnetic stirrer 4, stirring unit 1 is rotated in vessel 6. In this case, although stirring unit 1 rotates at a predetermined position, stirring unit 1 may be made to revolve while rotating the stirring unit 1 by revolving the rotating magnetic field generating section 42 (the same applies to other embodiments).

By rotation of stirring unit 1, fluid A retained or stagnated in mixing body 2 flows toward an outer periphery of mixing body 2 by receiving centrifugal force, and flows out of mixing body 2 from first through hole 22 of mixing element 21a which opens to an outer peripheral surface of mixing body 2 (see Fig. 2 and Fig. 4). On the other hand, fluid A in vessel 6 rotates and flows in a spiral manner toward the central lower portion where mixing body 2 is disposed, and flows into within hollow portion 24 through suction port 20α (see Fig. 2), which is the upper opening of hollow portion 24, from the upper portion of mixing body 2. Fluid A flowing into hollow portion 24 further receives a centrifugal force acting on stirring unit 1 (in this case, including a force by rotation of stirring unit 1, the same applies hereinafter), and flows into within mixing body 2 through first through holes 22 of mixing element 21b which open to an inner peripheral surface of hollow portion 24 (see Fig. 2 and Fig. 4). Then, fluid A flowing from one first through hole 22 at the inflow position passes through other first through hole 22 communicating with the one first through hole 22, further passes through still other first through hole 22 communicating with the other first through hole 22, and the like. Thus, fluid A flows within mixing body 2.

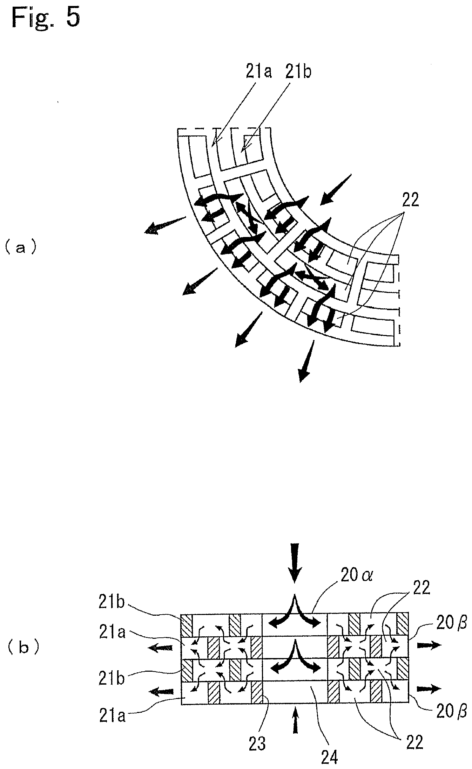

In this case, as shown in Fig. 5 at (a), fluid A flowing within the mixing body 2 passes through the plurality of first through holes 22 of mixing elements 21a and 21b, and flows in a substantially radial manner from the inner peripheral portion to the outer peripheral portion. At the time, fluid A is divided in an extending direction of the mixing elements 21a and 21b, and the divided fluids merge or combine.

In addition, mixing body 2 has a plurality of mixing elements 21a and 21b in a stacking direction, and is provided with a plurality of flow paths allowing fluid A (indicated by an arrow) to flow in the stacking direction of mixing elements 21a and 21b by the respective first through holes 22 communicating in the stacking direction. Therefore, when fluid A within mixing body 2 passes through first through holes 22, fluid A also flows in the stacking direction of mixing elements 21a and 21b as shown in Fig. 5 at (b), and fluid A is also divided in the stacking direction of mixing elements 21a and 21b to merge. Mixing elements 21a and 21b shown in Fig. 2 have different numbers of partition walls in the circumferential direction and in the radial direction from those shown in Fig. 5, but are not different in the function.

In this way, flow of the fluid A within mixing body 2 spreads not only in planar or two-dimensional division and merging in the extending direction of mixing elements 21a and 21b, but also in three-dimensional division and merging extending in the stacking direction of mixing elements 21a and 21b. The division and merging are carried out three dimensionally, whereby fluid A is highly mixed by repeating division, merging and the like. When mixing elements 21a and 21b are stacked one by one, planar and two-dimensional division and merging are performed, but even in this case, fluid A is repeatedly divided, merged, and mixed.

By the way, in the conventional stirring unit composed of rotating bodies such as bar-like or cross-shaped bodies, rotation energy is small at the center part of the stirring unit which is near the center of rotation. Even if a stirring unit provided with stirring blades on the rotating body (Patent Literature 1) applies a large rotational force to the fluid (A) in the upper part in the vessel (6), energy of rotation is still small at the central part of the stirring unit which is near the center of the rotation. Therefore, with these conventional stirring units, the fluid (A) in the center part in the vessel (6), in particular, the fluid (A) in the central upper part of the vessel (6) distant from the stirring unit only rotates in a small range in the radial direction. Therefore, the fluid (A) in the vessel (6) finally takes a long time to be uniformly stirred as a whole because the fluid (A) in the upper central portion in the vessel (6) is gradually stirred with the other portion.

On the other hand, as shown in Figs. 1 and 2, stirring unit 1 of this embodiment is provided with hollow portion 24 penetrating in the direction of rotation axis S at the central portion of mixing body 2. Therefore, fluid A in the upper central portion in vessel 6 is sucked into hollow portion 24 from the upper portion of mixing body 2 and mixed rapidly within mixing body 2.

As shown in Fig. 4, hollow portion 24 of mixing body 2 is formed by stacking second through holes 23 each having an opening area larger than that of each first through hole 22, and has a sufficiently large opening with respect to first through hole 22. Accordingly, as shown in Fig. 5 at (b), the flow resistance when the fluid A (indicated by an arrow) flows into hollow portion 24 is smaller than that of first through hole 22 opening on the upper surface of mixing body 2 . In addition, hollow portion 24 of mixing body 2 is located in the center of vessel 6 as shown in Fig. 1. Therefore, by rotation of stirring unit 1, fluid A in the upper central portion in vessel 6 opposed to hollow portion 24 can be easily introduced into hollow portion 24 via suction port 20α as shown in Fig. 5 at (b) without retention. Then, fluid A sucked into hollow portion 24 receives a centrifugal force by rotation of mixing body 2, flows into mixing body 2 and passes through each of first through holes 22, whereby as described above, fluid A is repeatedly divided and merged in the stacking direction and the extending direction of mixing elements 21a and 21b, respectively, to be highly mixed.

Hollow portion 24 of mixing body 2 is not always necessarily located at the center portion in vessel 6. If desired, by shifting the position of the center portion of stirring unit 1 from the center portion of vessel 6, hollow portion 24 may deviate from the center portion in vessel 6.

As described above, according to the stirring unit (1) having such a mixing body (2), the fluid (A) in the center portion of the vessel (6) at the center of rotation is also quickly stirred and the entire fluid can be stirred efficiently. As a result, it is possible to very shorten the time until the entire fluid (A) in the vessel (6) is uniformly stirred.

It should be noted that the present invention is not limited to this embodiment, and various modifications may be made within the scope of the gist of the present invention, and for example, the following modifications are available.

### (Modification 1 of mixing body 2)

First through hole 22 of mixing element 21 may be arranged nonlinearly in the extending direction of mixing element 21.

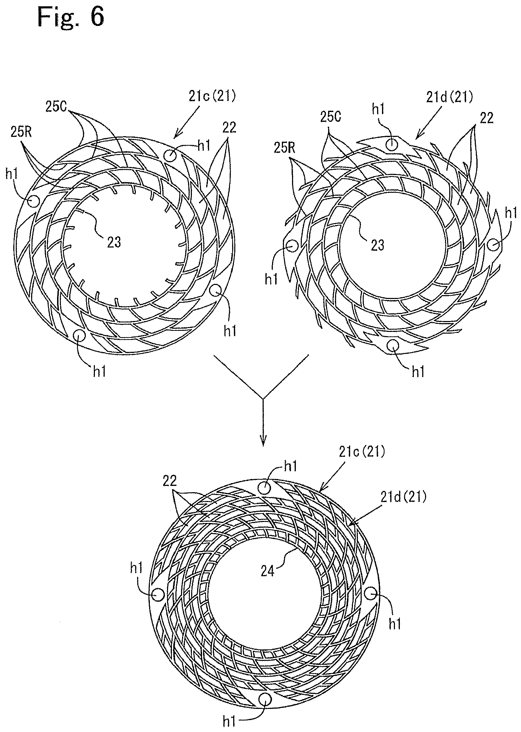

For example, as shown in Fig. 6, mixing elements 21c and 21d are formed to have partition walls 25R and 25C between first through holes 22, in which partition walls 25R curving in one direction from the center portion toward the outer side are formed to be in a substantially involute curve toward one direction side, and partitioned and connected by partition walls 25C extending continuously in the circumferential direction between these partition walls 25R. Partition walls 25C extending in the circumferential direction are formed concentrically around the center points of mixing elements 21c and 21d.

Thus, by disposing first through holes 22 in an involute shape (nonlinear shape), the flow resistance when the fluid flows through mixing body 2 can be made smaller than in the case where first through holes 22 are arranged in a linear line.

### (Modification 2 of mixing body 2)

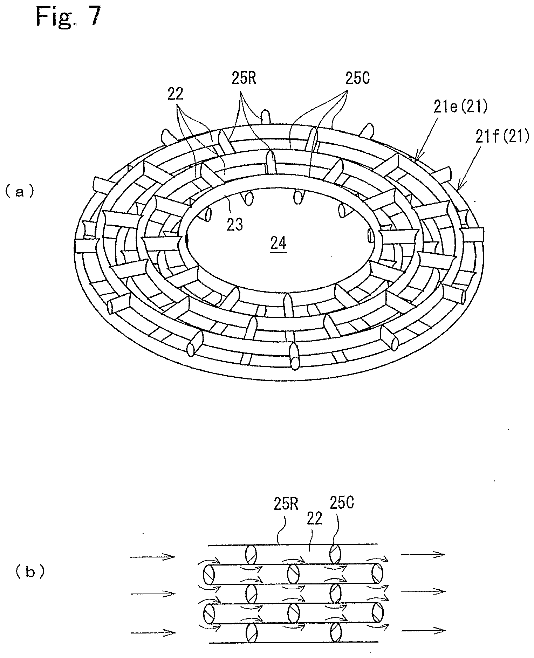

The above-described partition walls between first through holes 22 in mixing element 21 may be formed in a curved shape having no edge as seen in a cross sectional direction. For example, as shown in Fig. 7 at (a) and (b), elliptical or circular, or substantially vertically long elliptical shapes are formed in the cross-sectional shapes of partition walls 25R extending in the radial direction and partition walls 25C extending in the circumferential direction in mixing elements 21e and 21f.

The impact at the time of collision by the flow of fluid A in mixing elements 21e and 21f having partition walls 25R and 25C with such a cross-sectional elliptical shape can be reduced on outer surfaces of the partition walls as compared with mixing elements having partition walls with such a rectangular shape as seen in the cross-sectional direction. For example, in the field of cell cultivation such as production of fermented foods by yeast or the like, or cell cultivation such as liquid high density cultivation, even if a fluid A containing substances such as yeast and cells collides with the partition walls when the fluid A is uniformly stirred, the impact is suppressed, and good stirring may be performed without damaging the substances.

### (Modification 3 of mixing body 2)

The above-described partition wall 25 between first through holes 22 in mixing element 21 may be inclined, if desired.

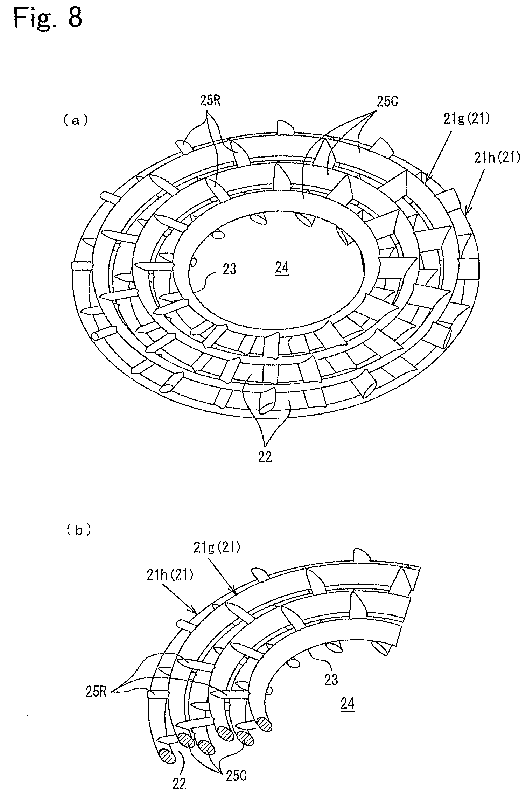

For example, as shown in Fig. 8 at (a) and (b), partition walls 25C extending in a circumferential direction in mixing elements 21g and 21h are inclined so as to spread toward the outer periphery as it goes upwardly, and partition walls 25R are inclined to one side in the right and left direction. The sectional shapes of partition walls 25R and 25C of mixing elements 21g and 21h shown in Fig. 8 are elliptical or substantially elliptical, but may be polygonal such as quadrangular.

Mixing elements 21g and 21h having such inclined partition walls 25R and 25C allow the flow of the fluid A within stirring unit 1 to be given directionality by the rotation of stirring unit 1. In the mixing elements of Fig. 8, it is possible to cause the fluid A to spirally flow downward or upward according to the direction of rotation of the stirring unit 1.

### (Modification 4 of mixing body 2)

The above-described first through holes 22 and second through holes 23 in the plurality of mixing elements 21 stacked in mixing body 2 may be configured to have different sizes for each mixing element 21.

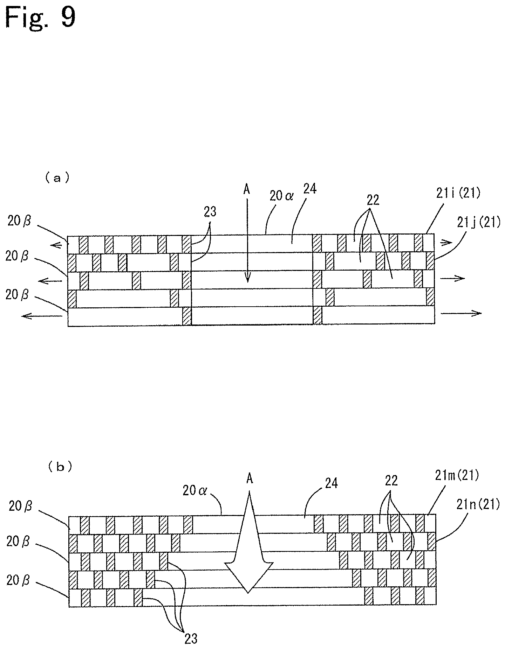

For example, as shown in Fig. 9 at (a), first through holes 22 in a plurality of stacked mixing elements 21i and 21j may be arranged such that first through holes 22 are larger toward the lower layer side.

With such a configuration, since the passage resistance when fluid A passes between first through holes 22 adjacent in the stacking direction within mixing body 2 decreases toward the lower layer side, it is possible within mixing body 2 to reduce the flow rate of fluid A flowing through the upper layer side and increase the flow rate of the fluid A flowing through the lower layer side.

Further, as shown in Fig. 9 at (b), second through holes 23 in a plurality of stacked mixing elements 21m and 21n are formed such that mixing elements 21m and 21n include second through holes 23 having a larger diameter toward the lower layer side and the inner diameter of a hollow portion 24 is configured to increase toward the lower layer side.

With such a configuration, the flow resistance of fluid A flowing in hollow portion 24 decreases toward the lower layer, so that the flow rate of fluid A flowing on the lower layer side can be increased.

### (Modification 5 of mixing body 2)

Mixing body 2 may be configured to be a single member provided with the above-described first through holes 22 and hollow portion 24 without using the structure stacked by the plurality of mixing elements 21. Mixing body 2 of such a single member may be easily produced by, for example, a 3D printer device. Further, as other mixing body 2, the above-described hollow portion 24 may be employed in a porous material member having continuous pores serving as first through holes 22.

### (Modification of base 3)

Although the above-described base 3 includes bar body 31 and annular portion 32 in this embodiment, the form of its shape and the like is not particularly limited as far as it does not fall over.

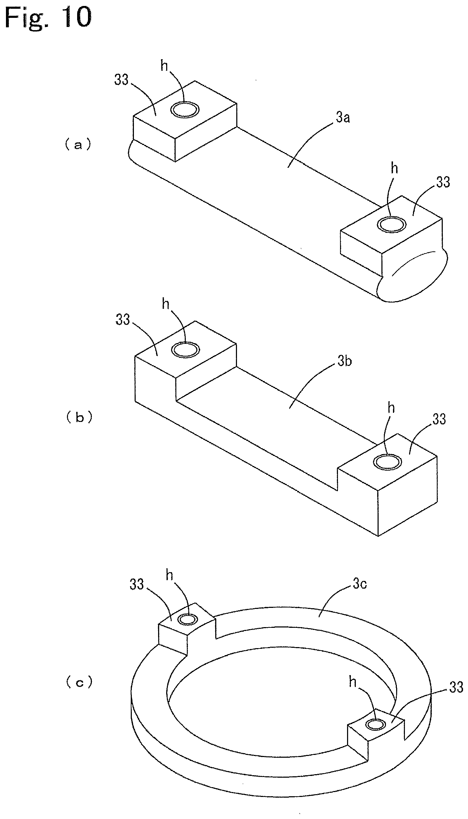

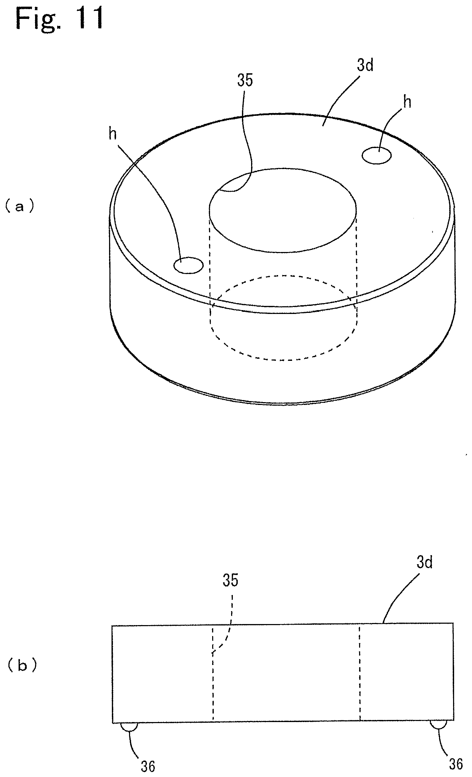

Such base 3 may employ bases in various forms, for example, a base 3a composed of a bar body having an elliptical columnar form shown in Fig. 10 at (a), a base 3b composed of a bar body having a flat rectangular columnar form shown in Fig. 10 at (b), a base 3c composed of an annular wheel body shown in 10 at (c), and a base 3d composed of a cylindrical body having a cylindrical form shown in Fig. 11. Raised portions (screw cylinder portion) 33 having screw holes "h" for mounting mixing body 2 are respectively disposed at both upper end portions of the bar bodies of bases 3a and 3b as shown in Fig. 10 at (a) and (b), and at two locations in a diameter direction of the upper surface of the wheel body of base 3c as shown in Fig. 10 (c), whereby a gap is formed between mixing body 2 and bases 3a, 3b and 3c respectively so that the flow of fluid A under the mixing body 2 is not stagnated.

In base 3d shown in Fig. 1 1, screw holes "h" for mounting mixing body 2 are provided at two positions of 180 degrees apart in an outer peripheral portion of an upper surface of the cylindrical body, and a vertical hole 35 penetrating the upper and lower surfaces is provided in the central portion. Even if mixing body 2 and base 3d are in contact with each other, a fluid A flowing in from the lower side of base 3d flows through first through holes 22 communicating with vertical hole 35 via hollow portion 24 of mixing body 2, and is delivered to an outer peripheral portion of mixing body 2 so that fluid A can be satisfactorily stirred without retention the flow of fluid A.

It is should be noted that, as with bases 3, 3a and 3b (see Fig. 3 at (a) and Fig. 10 at (a) and (b)), those having a structure capable of accommodating a bar magnet in a bar body extending in the longitudinal direction may accommodate the bar magnet horizontally housed within the bar body. In a base having such a cylindrical-shaped structure as base 3d (see Fig. 11) in which vertical hole 35 does not penetrate the lower surface thereof, a bar magnet may be horizontally accommodated in the lower part of the base.

Such a support protrusion 34 as shown in Fig. 3 at (b) and (c) are disposed on the bottom surfaces of bar shaped bases 3a and 3b, and two or more support projections 36 (see Fig. 11 at (b)) are provided at equal intervals, on bottom faces of wheel-shaped and cylindrical-shaped bases 3c and 3d.

### (Other embodiment 1)

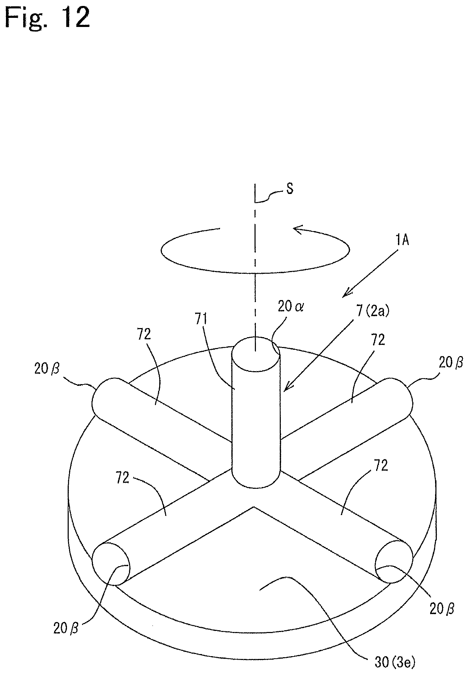

Fig. 12 shows a stirring unit 1A according to other embodiment 1 of the present invention. In stirring unit 1A, a mixing body 2 a is constituted by a tube body 7 forming open ends upward and sideways, and a base 3e is constituted by a disk body 30 holding tube body 7.

Tube body 7 (mixing body 2a) and disk body 30 (base 3e) may be connected by various connecting members such as a mating structure of unevenness or bonding with an adhesive. Tube body 7 includes a vertical tube 71 extending in the vertical direction and four lateral tubes 72 which are connected to a lower end of vertical tube 71 and extending sideways. Vertical tube 71 is disposed on a rotation axis S of stirring unit 1A, and the upper end opening thereof serves as a suction port 20α of fluid A. Lateral tubes 72 are arranged in a cross shape, and the respective side end openings serve as discharge ports 20β of fluid A. Accordingly, suction port 20α is located at a position on rotation axis S of stirring unit 1A, and discharge port 20β is located at a position outside rotation axis S than suction port 20α, that is, at a side outer side orthogonal to rotation axis S. Further, vertical tube 71 and lateral tube 72 communicate with each other, and its inside constitutes a flow path of fluid A. If desired, lateral tube 72 may be attached obliquely downward or obliquely upward with respect to longitudinal tube 71.

Though not shown in Fig. 12, magnets (5a and 5b of Fig. 3 at (a)) constituted similarly to the above-described embodiment are respectively accommodated in two places of base 3e at a 180 degree position on the outer peripheral portion of disc body 30. Further, an arcuate support protrusion (34 of Fig. 3 at (b)) in contact with a bottom surface of a vessel (6 of Fig. 1) protrudes from the center of a lower surface of disk body 30 in the same manner as in the above-described embodiment. In addition, a bar magnet may be housed as a magnet so that both end portions are positioned at the 180 degree position of base 3e.

A method of stirring fluid A by stirring unit 1A of this other embodiment 1 will be described hereinafter.

In the same manner as in the above embodiment shown in Fig. 1, as stirring unit 1A disposed at the bottom of vessel 6 is rotated by the rotating magnetic field from magnetic stirrer 4, the fluid within each lateral tube 72 is caused by the centrifugal force to flow out to the outside from discharge port 20β at the side end opening of lateral tube 72. Then, since fluid A within vertical tube 71 flows into each of lateral tubes 72, fluid A in the vicinity of the central upper portion in vessel 6 is caused to rotate and flow in a spiral toward tube body 7, and is drawn into vertical tube 71 from suction port 20α of the upper end opening of vertical tube 71. As a result, fluid A on the outer periphery of stirring unit 1A is mixed by being disturbed by fluid A flowing out from the discharge port 20β. Thus, fluid A in the upper central portion in vessel 6 is sucked from suction port 20α of the upper end opening of stirring unit 1A without retention and flows out from the discharge port 20β in the side end opening, so that it become possible to efficiently stir fluid A and shorten the time until entire fluid A is uniformly mixed.

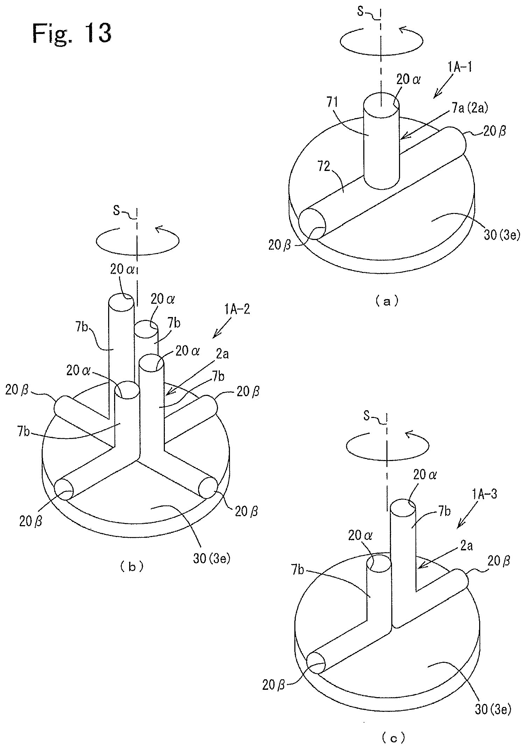

It is to be noted that lateral tube 72 may be formed to have an I-shape having openings (discharge ports 20β) at two lateral sides like the tube body 7a of the stirring unit 1A-1 shown in Fig. 13 at (a), or to employ lateral tubes of various forms opened at a plurality of lateral locations.

Though not shown in any drawings, the stirring unit (1) may be constituted by a pipe body with both ends opened as a mixing body (2) and magnets (5a and 5b) or magnetic substances which receive rotating magnetic fields at both end portions of the pipe body, respectively. In this example, the pipe body to be the mixing body (2) is placed horizontally with the longitudinal direction in the lateral direction, an opening serving as a suction port (20α) is disposed on an upper (or additionally lower) surface of the pipe body, and the openings at both ends of the pipe body may be discharge ports (20β). Further, magnets (5a and 5b) or magnetic substances may be attached to a lower surface or side surface of the both ends of the pipe body, or magnets (5a and 5b) or magnetic substances may be mounted on bases (3) which are attached to the lower surface, the side surface, or the like of both end portions of the pipe body. Accordingly, since such a stirring unit (1) is formed in an elongated shape, it is possible to use it by putting it into a vessel from an inlet of a vessel even if it has a narrow inlet.

In addition, like stirring units 1A-2 and 1A-3 shown in Fig. 13 at (b) and (c), the tube body constituting the mixing body 2a may be modified to an L-shaped tube body 7b having an upper end opening (suction port 20α) and a side end opening (discharge port 20β) such that a plurality of the L-shaped tube bodies 7b constitute a mixing body 2a by assembling the respective tube constituent parts in the longitudinal direction back to back. The respective heights of the upper end openings (suction ports 20α) of the plurality of L-shaped tube bodies 7b may be the same, or, as shown in Fig. 13 at (b) and (c), may be different. Thus, by arranging the respective heights of the plurality of suction ports 20α at different heights (see Fig. 13 at (b) and (c)), the fluid A at the respective positions of the upper part and the intermediate part of a central portion of vessel 6 may be sucked in at the same time, so that fluid A in the central portion can be more quickly stirred. In the L-shaped tube type mixed body 2a, each suction port 20α is disposed at a position near the rotation axis S of the stirring unit 1A-2 and 1A- 3, not at the position on the rotation axis S.

### (Other embodiment 2)

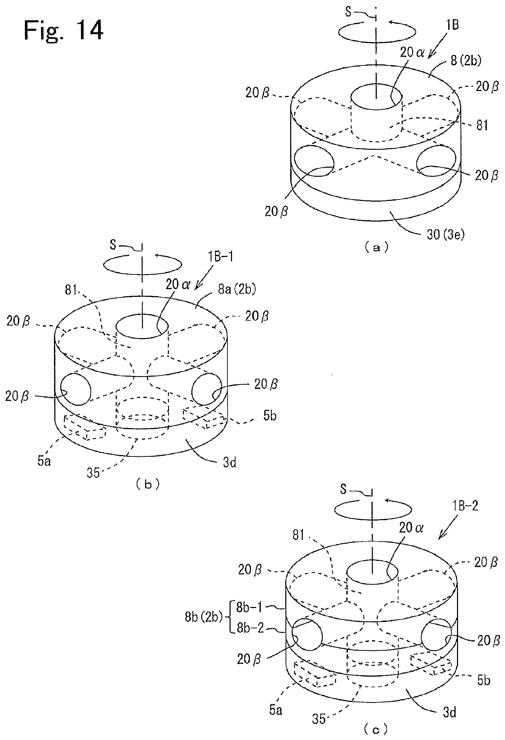

Fig. 14 at (a) - (c) shows stirring units 1B, 1B-1 and 1B - 2 as other embodiment 2 of the present invention. A mixing body 2b shown in Fig. 14 at (a) includes a cylindrical body 8 having openings (20α and 20β) on its upper and side surfaces respectively and communicating with the respective openings (20α and 20β) through an internal flow passage 81, and a base 3 is constituted by a disc body 30 having the same outer diameter as that of cylindrical body 8.

One opening on the upper surface of cylindrical body 8 is disposed in the center portion, and the opening portion of this upper surface serves as a suction port 20α of fluid A. Four openings on the side surface of cylindrical body 8 are arranged at equal intervals, and each opening on this side surface becomes a discharge port 20β of fluid A. Therefore, in cylindrical body 8 (mixing body 2a), suction port 20α is disposed at the position on rotation axis S of stirring unit 1B and discharge port 20β is located at a position outside rotation axis S than suction port 20α, that is, disposed at a lateral outside position orthogonal to rotation axis S. This cylindrical body 8 (mixing body 2b) has four internal flow passages 81 extending in the lateral direction and four discharge openings 20β, but this embodiment is not limited thereto. There may be provided a plurality of internal flow passages 81 and discharge openings 20β. Further, laterally extending internal flow passages 81 may be formed obliquely downward or obliquely upward with respect to internal flow passage 81 extending in the longitudinal direction from suction port 20α. Base 3e has a similar structure to that of the above-described other embodiment 1 (Fig. 12 and Fig. 13).

In addition, in the case where base 3 has a similar structure to that in Fig. 3, Fig. 10 or Fig. 11, opening 20α on the upper surface of cylindrical body 8 may be penetrated to the lower surface. In this case, fluid A may also be sucked from the lower surface of cylindrical body 8. In stirring units 1B-1 and 1B-2 shown in Fig. at 14 (b) and 14 (c), a base 3d is an example using base 3d shown in Fig. 11, and a vertical hole 35 of base 3d is arranged to communicate with a through hole in cylindrical body 8 (vertical flow path of internal flow path 81) serving as a mixing body 2b.

Further, in the case where cylindrical body 8 has an internal flow path 81 (a longitudinal flow path along a rotation axis S direction and a lateral flow path orthogonal to rotation axis S) communicating with side opening sections 20β, internal flow path 81 may be easily cleaned by dividing cylindrical body 8b if it is constituted by an upper cylindrical body 8b-1 and a lower cylindrical body 8b-2 which are configured to be vertically divided at a position of internal flow path 81 (lateral flow path) as shown in Fig. 14 at (c).

The stirring units 1B, 1B-1 and 1B-2 according to this other embodiment 2 can also achieve the same operation and effect as those of the above-described other embodiment 1.

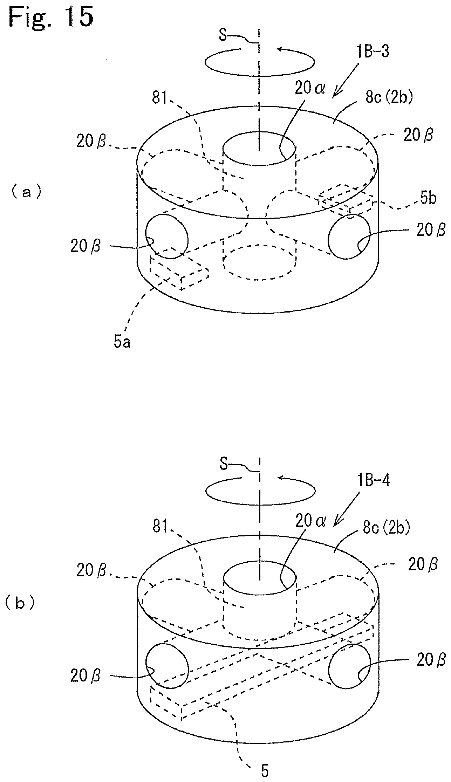

The stirring units 1B, 1B-1 and 1B-2 according to the other embodiment 2 have bases 3d and 3e as separate bodies from mixing body 2b, but mixing body 2b may have a cylindrical body integrated with bases 3d and 3e. In other words, mixing body 2b also serving as bases 3d and 3e may be formed by cylindrical body 8, and the magnets may be accommodated in the cylindrical body 8. For example, as in the case of a stirring unit 1B-3 shown in Fig. 15 at (a), when an opening on an upper surface of a cylindrical body 8c penetrates to a lower surface, a magnet of a cylindrical shape, a ring shape, a square shape or the like may be accommodated in cylindrical body 8c as a magnet 5 (note that Fig 15 at (a) is an example of square magnets 5a and 5b). As shown a stirring unit 1B-4 in Fig. 15 at (b), when an opening on an upper surface of a cylindrical body 8c does not penetrate the lower surface, a bar magnet other than the foresaid magnet also may be accommodated in cylindrical body 8 as a magnet 5.

### (Other Embodiment 3)

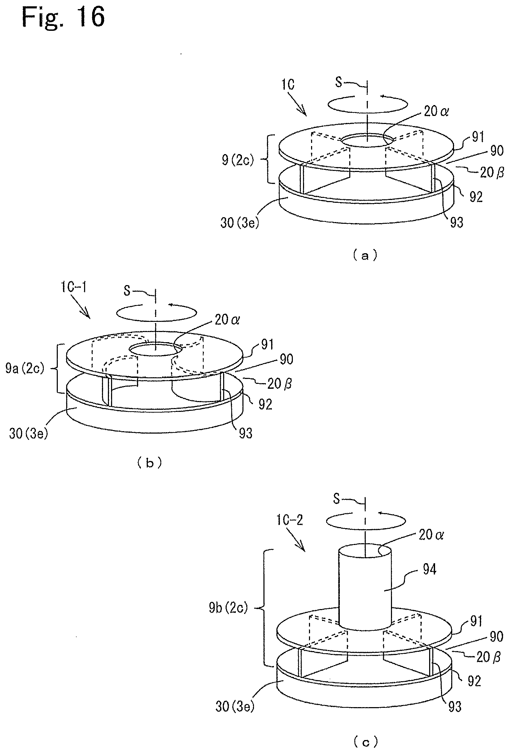

Fig. 16 at (a) - (c) shows perspective views of stirring units 1C, 1C-1 and 1C-2 as other embodiment 3 of the present invention. As shown in Fig. 16 at (a), a mixing body 2c is composed of a structure body 9 in which a disk-shaped annular plate 91 having a through hole in the center and other disk 92 are connected by a plurality of connection walls 93, openings (suction port 20α and discharge port 20β) are disposed on an upper surface and a side surface of structure body 9, and a base 3e is constituted by a disk member 30 having the same outer diameter as the outer diameter of structure body 9. With such a configuration also, since the outer peripheral end of a gap 90 between annular plate 91 and circular plate 92 separated by the plurality of connection walls 93 becomes discharge port 20β of a fluid (A), by rotation of stirring unit 1C, the fluid (A) within a vessel (6) may be sucked from the opening (suction port 20α) on the upper surface, and discharged from the side opening (discharge port 20β) so as to be stirred. In structure body 9 as mixing body 2c, four connection walls 93 connecting annular plate 91 and circular plate 92 are provided, but the number is not limited to this and may be smaller or larger than four. Further, like a stirring unit 1C-1 shown in Fig. 16 at (b), connection wall 93 may have an involute shape (nonlinear shape) in a plan view.

In a stirring unit 1C-2 of this other embodiment 3 as shown in Fig. 16 at (c), mixing body 2c may be a structure body 9b in which a pipe shaped suction tube 94 is connected to annular plate 91. In this case, one end of suction tube 94 connected to annular plate 91 opens to an upper surface and becomes suction port 20α of the fluid (A). It should be noted that suction tube 94 may be in the form of a trumpet (not shown in drawings).

### (Other Embodiment 4)

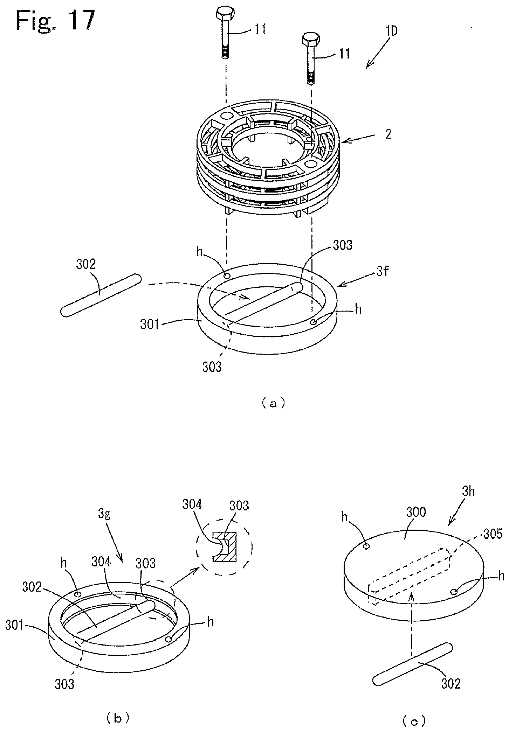

Fig. 17 at (a) shows a stirring unit 1D according to other embodiment 4 of the present invention. For example, as shown in Fig. 17 at (a), a base 3f is constituted by fitting a bar-shaped stirrer 302 into a support base (holding body) 301 in a fittable manner.

The bar-shaped stirrer 302 may be constituted by, for example, a rotator holding magnets or magnetic substances. Base 3f is provided with attachment holes 303 at a pair of positions of 180 degrees on an inner surface of support base 301 formed in an annular shape, and is constituted by fitting and fixing both end portions of bar-shaped stirrer 302 to attachment holes 303. Screw holes "h" are disposed at a pair of places in a diametrical direction on an upper surface of support base 301, and a mixing body 2 is attached by screwing screws 11 inserted into mixing body 2 into the screw holes "h". Thereby, for example, it is possible to easily construct a stirring unit having mixing body 2 by using bar-shaped stirrer 30 of the existing product.

A base 3g shown in Fig. 17 at (b) as another example of base 3 is configured such that a recessed groove 304 continuous in a circumferential direction is formed on an inner surface of a support base 301 so that bar-shaped stirrer 302 can be easily fitted to support 301. Further, a base 3h shown in Fig. 17 at (c) may be configured such that a support base 300 is formed in a disk shape and a bar-shaped stirrer 302 is fitted and fixed in a fitting groove 305 provided in a bottom surface of the disk. Here, screw holes "h" of screws 11 for attaching mixing body 2 are provided on upper surfaces of support bases 301 and 300 shown in Fig. 17 at (b) and 17 (c).

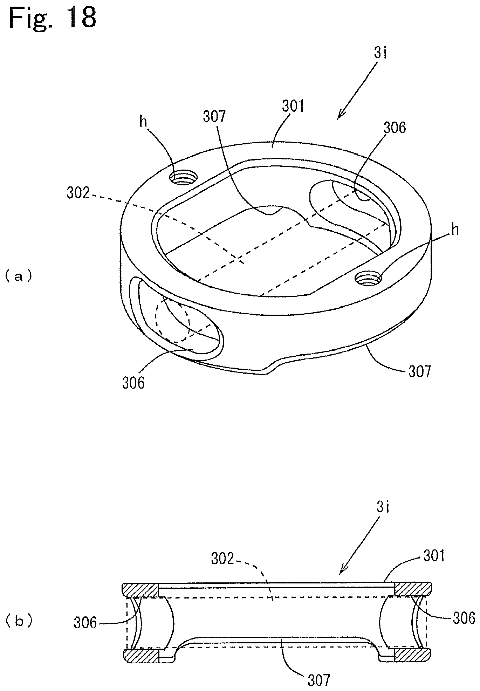

Further, in a base 3i shown in Fig. 18 at (a) and (b), a supporting base 301 is formed into an annular shape, and elongated holes 306 extending in its circumferential direction are provided at a pair of positions of an inner side at 180 degrees, whereby base 3i is constituted by fitting and fixing two ends of bar-shaped stirrer 302 into the respective elongated holes 306. In this case, in elongated holes 306 into which bar-shaped stirrer 302 is fitted, there is formed a gap so as to communicate an inner side and an outer side of support base 301, so that a centrifugal force acting on stirring unit 1D passes fluid A on the inner side of support base 301 through the gap toward the outer side of support base 301, so that fluid A does not retain on the inner side of support base 301. Further, on the lower surface of the support base 301, notches 307 are provided in an opposed portion between the pair of opposed long holes 306. These notches 307 also make it possible to prevent fluid A from retaining on the inner side of support base 301. Threaded holes "h" of screws 11 for attaching mixing body 2 to two positions in the diameter direction is provided on an upper surface of support base 301.

In addition to support bases (300 and 301) shown in Figs. 17 and 18, it is also possible to use a configuration that allows attachment of bar-shaped stirrer 302.

### (Other Embodiment 5)

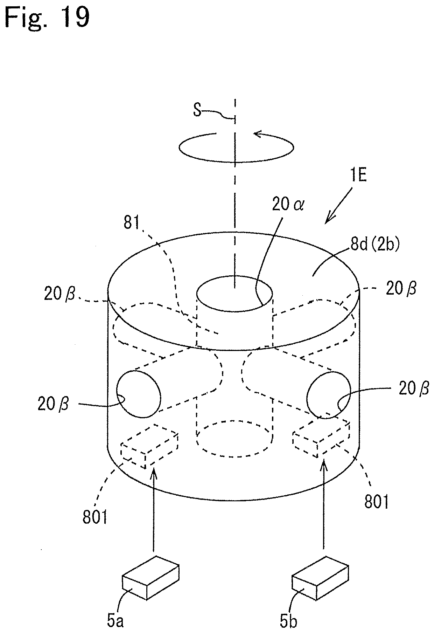

Fig. 19 is a perspective view of a stirring unit 1E according to other embodiment 5 of the present invention. For example, in Fig. 19, magnets 5a and 5b are fixed in a manner to be fitted. A pair of fitting grooves 801 for fitting and fixing magnets 5a and 5b are provided at a pair of positions in a diameter direction of a lower surface of a cylindrical body 8d constituting stirring unit IE. Thereby, stirring unit IE can be easily constructed. Further, for example, it is possible to easily adjust the magnetic force of stirring unit 1E by selecting and using magnets 5a and 5b having different magnetic forces, depending on its purpose, use, and the like.

In the stirring unit 1E, it is preferable to use magnets 5a and 5b coated with a resin. For example, when cylindrical body 8d is made of a fluororesin, magnets 5a and 5b also coated with a fluororesin is used. The shapes of magnets 5a and 5b are not limited to the square type as shown in Fig. 19, and various shapes such as a cylindrical type may be used. Cylindrical body 8d has substantially the same structure as that of cylindrical body 8c shown in Fig. 15 at (a) as a structure of a mixing body 2b, but it is not limited to this, and it can be formed in various mixed structures. Cylindrical body 8d also has the function of a base (3) provided with magnets 5a and 5b, and has the functions of mixing body 2b and base 3 integrally. If desired, mixing body 2b and base 3 may be formed separately so as to be assembled, and have a fitting groove 801 in which magnets 5a and 5b are fitted and attached. Also, magnets 5a and 5b are not limited to the two, but may be constituted by one bar magnet, and cylindrical body 8d may be provided with a fitting groove which can be fitted and fixed by the bar magnet in a lateral direction.

Holding rings such as annular shapes may be provided on magnets 5a and 5b respectively, and the respective holding rings may be fitted and fixed on the end portions of tube body 7 as shown in Fig. 12. In this case, magnets 5a and 5b provided with the holding wheels may be provided on the four lateral tubes 72 or only on the two opposed lateral tubes 72. In this case, stirring unit 1A is constituted with tube body 7 and magnets 5a and 5b with holding rings, so that the base 3e becomes unnecessary. Likewise, for example, magnets 5a and 5b with holding rings may be fitted and fixed to both end portions of the tube body 7a as shown in Fig. 13 (a) so that the base 3e is not required.

### (Other embodiments)

According to another embodiment of the present invention, the mixing body portion in the above embodiments may be modified to be directly connected to a motor to constitute a mixing body for stirring a fluid in a vessel.

That is, there is configured a mixing body (see Fig. 2, Fig. 6, Fig. 7, Fig. 8, Fig. 9 and the like) for stirring the fluid contained in the vessel by rotating around the rotation axis, wherein a suction port and a discharge port for the fluid are provided on a surface of the above-described mixing body, one or two or more holes connecting the suction port and the discharge port are provided within the mixing body, the suction port is disposed at a position on the rotation axis or a position closer to the rotation axis than the discharge port, and the discharge port is disposed at a position outside the rotation axis than the suction port (for example, a position outside in a radial direction orthogonal to the rotation axis).

Further, there is provided a mixing body for stirring a fluid contained in a vessel by rotating around the rotation axis (see Figs. 12 to 16, 18, 19, and the like), wherein the mixing body includes one or two or more flow paths, one end side opening of the flow path constitutes a fluid suction port, other end side opening of the flow path constitutes a fluid discharge port, the suction port is disposed at a position on the rotation axis or at a position close to the rotation axis, and the discharge port is disposed at a position outside the rotation axis than the suction port (for example, a position outside in a radial direction orthogonal to the rotation axis).

Further, it is possible to constitute a stirring device comprising the above mixing body.

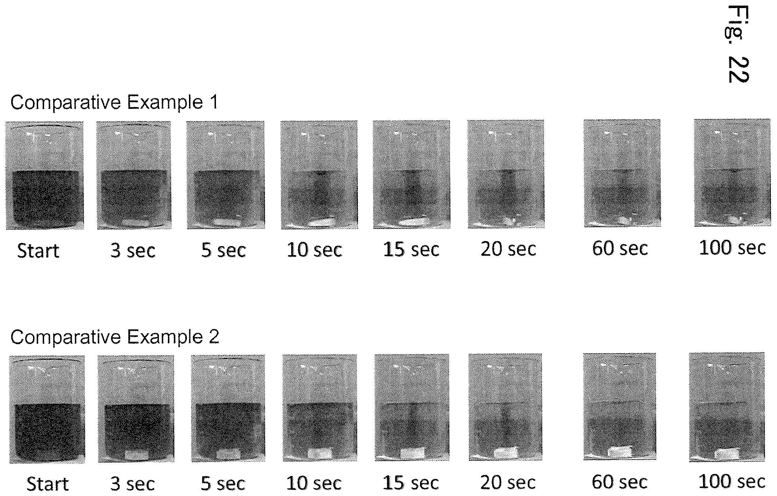

Specifically, as the stirring device, there is a stirring device shown in Fig. 1 in which shaft portion 44 of motor 45 is directly connected to the mixing body through the upper surface of device main body 41 and the bottom surface of vessel 6. Also in the stirring device shown in Fig. 22, the shaft portion of the motor of magnetic stirrer 4 is directly connected to the mixing body by penetrating the upper flat surface of the main body and the lid 6a. In these cases, the rotating magnetic field generating section 42, the magnets 5a and 5b, or the base 3 may be omitted. In the stirring device as shown in Fig. 1, a predetermined sealing mechanism is provided between the bottom of vessel 6 and the shaft portion 44.

Even with the mixing body and stirring device of the above-described other embodiments, like each of the above-described embodiments, the fluid (A) in the central portion of the vessel (6) at the center of rotation is also rapidly stirred to efficiently stir the entire fluid (A). As a result, it is possible to very shorten the time until the entire fluid (A) in the vessel (6) is uniformly stirred.

### (Example)

Next, in order to ascertain the effect of stirring by the stirring unit of the present invention, the following decolorizing experiment was carried out.

For the decolorization experiment, an iodine decolorization reaction was used. Specifically, a beaker (vessel 6) containing an iodine solution (fluid A) was placed on a magnetic stirrer (4), the stirring unit (1) of the example was placed in the bottom of the beaker to stir the iodine solution, and the time for decolorization until the iodine solution became transparent was measured after adding an aqueous sodium thiosulfate solution to the stirring iodine solution. The time until the decolorization may be regarded as the time during which the sodium thiosulfate aqueous solution is uniformly mixed with the iodine solution as a whole, that is, the time when the entire fluid A is uniformly mixed, and the stirring effect of the stirring unit was verified from the length of the time until the decolorization.

The specifications of each solution used in the decolorization experiment are as follows;
Iodine solution: 20 cc of 0.05 mol / L iodine solution (0.1N)
Sodium thiosulfate aqueous solution: 4 cc of aqueous solution prepared by dissolving 37.5g of sodium thiosulfate in 200 cc of water

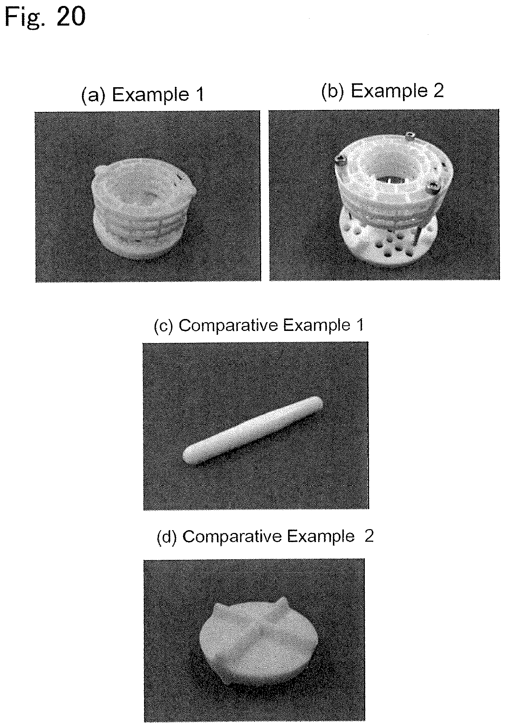

As to stirring unit (1) of Examples 1 and 2, there were used those shown in the photographs of Fig. 20 at (a) "Example 1" and Fig. 20 at (b) "Example 2".

A stirring unit (1) of Example 1 is shown in Fig. 20 at (a), and its mixing body (2) has a structure as shown in Fig. 2 (a pair of first through holes 22 of mixing elements 21a and 21b are arranged in a radial direction.), and its base (3) has a disk structure in which screw cylinder portions (33) are provided at a pair of positions on an upper surface of disk body 30 of Fig. 12. The specification size of the stirring unit 1 of Example 1 is as follows.

The base (3) has a disk portion with a diameter (outer diameter) of 40 mm and a height of 14 mm, and there is a gap of 5 mm between the base (3) and the mixing body (2). The mixing body (2) is formed by alternately stacking three sets (using a total of twelve mixing elements) each of which is consisting of a pair of mixing elements (21a) each having a height of 1 mm overlapped with each other and a pair of mixing elements (21b) each having a height of 1 mm overlapped with each other, and has a diameter (outer diameter) of 37.5 mm, an inner diameter of the hollow portion of 19 mm and a height of 12 mm.

A stirring unit (1) of Example 2 is shown in Fig. 20 at (b), and its mixing body (2) has a structure as shown in Fig. 2 (three first through holes 22 in each of the mixing elements 21a and 21b are arranged in a radial direction.), and its base (3) has a disk structure in which screw holes (h) are provided at three positions on an upper surface of disk body 30 of Fig. 12 and a plurality of through holes vertically penetrating through the base are disposed to reduce its weight. The specification size of the stirring unit (1) of Example 2 is as follows.

The base (3) has a disk portion with a diameter (outer diameter) of 60 mm and a height of 15 mm, and a gap of 20 mm is provided between the disk (3) and the mixing body (2). The mixing body (2) has a three-layered structure formed by alternately stacking three sets (using a total of eighteen mixing elements) each of which is consisting of three mixing elements (21a) each having a height of 1 mm overlapped with each other and three mixing elements (21b) each having a height of 1 mm overlapped with each other, and has a diameter (outer diameter) of 54 mm, an inner diameter of the hollow portion of 27 mm and a height of 18 mm. The space between mixing body (2) and base (3) has a height of 20 mm.

As Comparative Example 1, a stirring bar (trade name "Rotor" manufactured by As One Corporation) made of a bar body shown in the photograph of Fig. 20 at (c) was used. The stirring bar of Comparative Example 1 is a substantially cylindrical shape having a length of 60 mm and a width (diameter) of 8 mm.

Further, as Comparative Example 2, there was used a stirring disk (trade name "Crosshead rotator double" manufactured by As One Corporation) having protrusions arranged in a cross shape on upper and lower surfaces of the disk shown in the photograph of Fig. 20 at (d). The stirring disk of Comparative Example 2 is a substantially disk-shaped one having a diameter of 40 mm and a height of 14 mm (the thickness of the disk is 5.6 mm, and the height of each of the upper and lower projections is 4.2 mm).

As a result of the above decolorization experiments, the time until decolorization was 20 seconds in the stirring unit (1) of Example 1 and 5 seconds in the case of the stirring unit (1) in Example 2, whereas in the case of the stirring bar of Comparative Example 1 took 100 seconds, and the stirring disk of Comparative Example 2 took 70 seconds. That is, according to the stirring unit (1) of the present embodiment, it is possible to complete the mixing of the entire fluid A uniformly in a short time of 1/3.5 or less compared with the stirring unit of the comparative example, so that high stirring performance and high mixing performance could be confirmed.

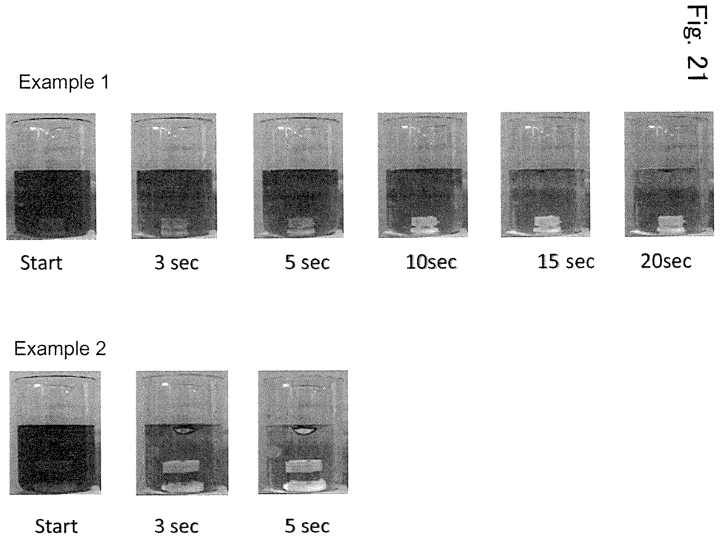

Further, the state of stirrings was observed as shown in the photograph of Fig. 21 (Example 1 and Example 2). That is, in the stirring units (1) of Example 1 and Example 2, the solution in the beaker changed to be transparent from the bottom to the top of the beaker as a whole. Accordingly, it can be found that the stirring of a fluid (A) is being performed by the stirring unit (1) which sucks the solution at the center upper part in the beaker from the upper part of mixing body (2) into the hollow part and mixes the same within the mixing body (2) to flow so as to discharge to the outer periphery side of the mixing body (2).

On the other hand, in the stirring units of Comparative Example 1 and Comparative Example 2, as shown in the photograph of Fig. 22 (Comparative Example 1 and Comparative Example 2), the solution on the outer peripheral side in the beaker changed transparently from the bottom portion of the beaker to the upper portion, and then the solution in the central part with each stirring unit changed to transparent from bottom to top of the beaker. Accordingly, it can be found that the energy of rotation is small at the center part of the stirring unit which is the center of rotation and particularly the movement of the solution is dull at the central upper part most distant from the stirring unit whereby a long time is required for the fluid A in the central portion and the central upper portion to be mixed with the fluid A in the other portion and to be uniformly mixed as a whole.

### REFERENCE SIGNS LIST

1; stirring unit
2; mixing body
3; base
4; magnetic stirrer
5a and 5b; magnet
6; vessel

## Claims

1. A stirring unit for stirring a fluid contained in a vessel, comprising:
a mixing body for stirring the fluid by rotating around a rotation axis, and
a magnet or a magnetic substance for receiving a rotating magnetic field for rotating the mixing body,
wherein a suction port and a discharge port for the fluid are provided on a surface of the mixing body,
wherein one or two or more holes connecting the suction port and the discharge port are provided within the mixing body,
wherein the suction port is disposed at a position on the rotation axis or at a position closer to the rotation axis than the discharge port, and
wherein the discharge port is disposed at a position outside the rotation axis than the suction port.

2. A stirring unit according to claim 1, wherein the mixing body is fitted and fixed with the magnet, the magnetic substance, or a rotator that holds the magnet or the magnetic substance.

3. A stirring unit according to claim 1 or 2, wherein a hollow portion extending in a rotation axis direction and communicating with the suction port is provided in a central portion of the mixing body.

4. A stirring unit according to any one of claims 1 to 3, wherein a part or the whole of a hole within the mixing body is arranged so as to directly communicate a fluid with a hole adjacent in a direction of the rotation axis and to divide the fluid in the rotation axis direction and the radial direction, and the suction port and the discharge port are connected to each other.

5. A stirring unit according to any one of claims 1 to 4,
wherein the mixing body is constituted by a stacked structure in which a plurality of mixing elements are stacked in a direction of the rotation axis,
wherein the mixing element has a plurality of first through holes serving as holes within the mixing body, and
wherein in the mixing body, the mixing elements are arranged such that a part or the whole of the first through hole in one of the mixing elements partly overlaps with the first through hole of its adjacent one of the mixing elements by shifting their positions, and communicably communicates the fluid with the first through hole of the adjacent one of the mixing elements so as to divide the fluid in a stacking direction and an extending direction of the mixing elements.

6. A stirring unit according to claim 5,
wherein the mixing elements have second through holes larger than the first through holes, and
wherein in the mixing body, the second through holes communicate with each other in a stacking direction to form a hollow portion for allowing a fluid to flow into the mixing body.

7. A stirring unit according to claim 5 or 6,
wherein the mixing element has partition walls that partition the plurality of first through holes respectively, and
wherein the partition wall is formed in a circular shape or an elliptical shape as seen in a cross section.

8. A stirring unit according to any one of claims 5 to 7,
wherein the mixing body is fixed to a base having the magnet or the magnetic substance, in which the plurality of mixing elements are disassemblably fixed in a stacked state by a fixing member.

9. A stirring unit according to any one of claims 1 to 4, wherein the mixing body is constituted by a single member.

10. A stirring unit for stirring a fluid contained in a vessel, comprising:
a mixing body for stirring the fluid by rotating around a rotation axis, and
a magnet or a magnetic substance for receiving a rotating magnetic field for rotating the mixing body, wherein
the mixing body has one or more flow paths,
a one end side opening of the flow passage constitutes a suction port for the fluid,
an opening on other end side of the flow path constitutes a discharge port for the fluid,
the suction port is disposed at a position on the rotation axis or at a position close to the rotation axis, and
the discharge port is disposed at a position outside the rotation axis than the suction port.

11. A stirring unit according to any one of claims 1 to 10, further comprising a base having the magnet or the magnetic substance.

12. A stirring unit according to claim 11, wherein the base is configured by fitting and fixing the magnet, the magnetic substance, or a rotator that holds the magnet or the magnetic substance, in a holding body.

13. A stirring unit according to claim 11 or 12, wherein the mixing body and the base are integrally formed.

14. A stirring unit according to claim 10, wherein the mixing body is composed of a tube body constituting the flow path or a structure provided with the flow path within the structure.

15. A stirring unit according to any one of claims 11 to 13, wherein the base is formed by a bar body.

16. A stirring unit according to claim 15, further comprising a protrusion at a center position of a bottom of the bar body.

17. A stirring unit according to claim 15 or 16, wherein the base further includes an annular portion connected to both end portions of the bar body.

18. A stirring unit according to claim 17, further comprising a protrusion at a bottom of the annular portion.

19. A stirring unit according to claim 17 or 18, wherein the magnet or the magnetic substance is provided in the annular portion.

20. A stirring unit according to any one of claims 1 to 19, wherein a surface layer of the stirring unit is formed of a fluororesin.

21. A stirring device comprising the stirring unit according to any one of claims 1 to 20, and a rotating magnetic field generating unit for generating a rotating magnetic field for rotating the stirring unit.

22. A stirring device according to claim 21, wherein the rotating magnetic field generating unit is configured to revolve the stirring unit while self rotating.

23. A stirring method for stirring a fluid, comprising the steps of;
disposing the stirring unit according to any one of claims 1 to 20 at a bottom portion in a vessel,
rotating the stirring unit at the bottom portion within the vessel for containing the fluid,
sucking the fluid within the vessel through the suction port by the stirring unit,
passing the fluid within the stirring unit, and
flowing out the fluid from the discharge port of the stirring unit into the vessel to stir the fluid.

24. A stirring method according to claim 23, further comprising the step of revolving the stirring unit while self rotating the same.

25. A cell cultivation method using the stirring unit according to any one of claims 1 to 20, wherein the fluid in the vessel is set as a cell cultivation medium, and the cell cultivation liquid is stirred with the stirring unit.

26. A cell cultivation method according to claim 25, further comprising the step of stirring a cell cultivation solution while controlling the temperature of the cell cultivation solution or the cultivation environment.

27. A reaction promoting method using a stirring unit according to any one of claims 1 to 20, wherein the fluid in the vessel is set as a reaction solution, and stirring the reaction solution with the stirring unit to promote the reaction.

28. A reaction promoting method according to claim 27, further comprising the step of stirring the reaction solution while controlling the temperature of the reaction solution or the reaction environment.

29. A method of assembling the stirring unit according to any one of claims 5 to 8, comprising the steps of;
forming the mixing body,
fixing the mixing body to the magnet or the base having the magnetic substance, wherein
the step of forming the mixing body includes a step of aligning and stacking a plurality of the mixing elements to form a mixing body,
the step of fixing the mixing body to the base includes a step of fixing the mixing body to the base by penetrating the mixing body in a stacking direction by a fixing member.
